# EUROPEAN PATENT APPLICATION

(11) **EP 2 135 856 A1**
(43) Date of publication of application: **23.12.2009**
(21) Application number: 08739389.8
(22) Date of filing: 25.03.2008
(51) Int. Cl.: C07C 51/285

(54) **PROCESS FOR PRODUCING 1,2,3,4-BENZENETETRACARBOXYLIC ACID**

(30) Priority: 19.04.2007 JP 2007110118
(71) Applicant: JFE Chemical Corporation, Tokyo 111-0051 (JP)
(72) Inventor: KITAMURA, Naoyuki, Tokyo 111-0051 (JP)
(74) Representative: Grünecker, Kinkeldey, Stockmair & Schwanhäusser Anwaltssozietät
(86) International application number: PCT/JP2008/056272
(87) International publication number: WO 2008/132929

(57) **Abstract**

A method for preparation of 1,2,3,4-benzenetetracarboxylic acid includes the step of oxidizing 1,2,3,4,5,6,7,8-octahydrophenanthrene with potassium permanganate. This method for preparation is superior to conventional methods because raw materials are easily available, special facilities are not required, and fewer reaction steps are needed. The resulting 1,2,3,4-benzenetetracarboxylic acid is useful as a raw material for functional polymers or as an intermediate of various chemicals.

## Description

### Technical Field

The present invention relates to a method for preparation of 1,2,3,4-benzenetetracarboxylic acid. More particularly, the present invention relates to a method for preparation of 1,2,3,4-benzenetetracarboxylic acid, which is useful as a raw material for various polymers, including functional polymers, such as polyimide resin, or as an intermediate of various chemicals and which is expected to be utilized as a curing agent for epoxy resin or as an additive agent for polyesters or to be derivatized by an esterification reaction or an amidation reaction.

### Background Art

Various methods for preparation of 1,2,3,4-benzenetetracarboxylic acid have been proposed. However, each of these methods has problems or issues to be addressed. Scale-up from the laboratory scale is particularly difficult.

For example, in accordance with Ber. 1884, 17, 2517 and Ber. 1888, 21, 904, 1,2,3,4-tetramethylbenzene is oxidized to synthesize 1,2,3,4-benzenetetracarboxylic acid. Also in Pharmaceutical Society of Japan 1953, 73, 928, 1,2,3,4-tetramethylbenzene prepared by synthesis is oxidized with potassium permanganate to synthesize 1,2,3,4-benzenetetracarboxylic acid.

However, in these preparation methods, a raw material 1,2,3,4-tetramethylbenzene is difficult to obtain; it requires difficult recovery and purification of an infinitesimal amount of the component contained in petroleum or synthesis involving complicated organic reactions.

In accordance with J. Chem. Soc. 1910, 97, 1904, 1,4-dimethylnaphthalene is oxidized with 40% concentrated nitric acid at a reaction temperature in the range of 170°C to 180°C under pressure to synthesize 1,2,3,4-benzenetetracarboxylic acid. In accordance with U.S. Patent No. 3,350,443, 1,2,3,4,5,6,7,8-octahydrophenanthrene is oxidized with concentrated nitric acid at a reaction temperature of 150°C under pressure or a reaction temperature in the range of 160°C to 165°C in a dibromobenzene solvent to synthesize 1,2,3,4-benzenetetracarboxylic acid.

However, both of the preparation methods need special facilities, facilities with acid resistance and high-pressure resistance, and produce a large amount of nitrogen oxide. These preparation methods therefore have problems also from a safety and environmental standpoint.

In accordance with DE 1,183,068, 1,2,3,4,5,6,7,8-octahydrophenanthrene is oxidized with oxygen in a butyrolactone solvent at a reaction temperature of 210°C and a pressure of 20 kgf/cm² to synthesize 1,2,3,4-benzenetetracarboxylic acid.

However, this preparation method using liquid phase oxygen oxidation needs special facilities, such as facilities with high-pressure resistance, and use of a high-pressure oxygen gas, which causes safety problems.

In accordance with J. Am. Chem. Soc. 1933, 55, 4305, J. Am. Chem. Soc. 1939, 61, 288, J. Am. Chem. Soc. 1952, 74, 116, and Macromolecules 2002, 35, 8708, 1,4-naphthalenedicarboxylic acid is oxidized with potassium permanganate to synthesize 1,2,3,4-benzenetetracarboxylic acid.

However, these preparation methods require a several-step synthetic reaction to prepare the raw material 1,4-naphthalenedicarboxylic acid. Furthermore, the purification and isolation of 1,2,3,4-benzenetetracarboxylic acid after reactions are difficult and result in a low yield.

In accordance with Bull. Chem. Soc. Jpn. 1968, 41, 1, 265, a raw material 1,3-cyclohexadiene is subjected to a three-step process involving a Diels-Alder reaction with maleic anhydride, liquid phase nitric acid oxidation, and oxidative dehydrogenation with bromine to synthesize 1,2,3,4-benzenetetracarboxylic acid. In accordance with Macromolecules 2002, 35, 8708, after 1,3-cyclohexadiene is synthesized using cyclohexene as a starting material, the same process as described above including the Diels-Alder reaction with maleic anhydride is performed to synthesize 1,2,3,4-benzenetetracarboxylic acid.

However, both of these preparation methods require a large number of steps, need acid-resistant special facilities in the nitric acid oxidation, and produce a large amount of nitrogen oxide, causing problems from a safety and environmental standpoint.

Accordingly, it is an object of the present invention to provide a method for preparation of 1,2,3,4-benzenetetracarboxylic acid, which is useful as a raw material for functional polymers or as an intermediate of various chemicals, using raw materials that can be obtained more easily than those used in conventional methods, without using special facilities, in a smaller number of reaction steps.

### Disclosure of Invention

The object described above can be achieved by the present invention described below.

The present invention provides a method for preparation of 1,2,3,4-benzenetetracarboxylic acid that includes the step of oxidizing 1,2,3,4,5,6,7,8-octahydrophenanthrene with potassium permanganate.

In this method for preparation, the oxidation is preferably performed in an aqueous reaction medium. In this method for preparation, the oxidation is more preferably performed at a mass ratio of potassium permanganate to the reaction medium in the range of 1:2 to 1:50.

In any one of the methods for preparation described above, the oxidation is preferably performed under alkaline conditions.

In any one of the methods for preparation described above, the oxidation is preferably performed at a temperature in the range of 10°C to 120°C.

In any one of the methods for preparation described above, the oxidation is preferably performed at a molar ratio of 1,2,3,4,5,6,7,8-octahydrophenanthrene to potassium permanganate in the range of 1:8 to 1:30.

Preferably, any one of the methods for preparation described above further includes the step of extracting 1,2,3,4-benzenetetracarboxylic acid with acetic acid and purifying 1,2,3,4-benzenetetracarboxylic acid after the oxidation reaction.

### Brief Description of Drawings

[Fig. 1] Fig. 1 is the IR spectrum of 1,2,3,4-benzenetetracarboxylic acid prepared in Example 1.
[Fig. 2] Fig. 2 is a graph illustrating the reaction yield as a function of reaction time in Examples 1 to 5.

### Best Modes for Carrying Out the Invention

The present invention will be further described in the following preferred embodiments. In the present invention, 1,2,3,4,5,6,7,8-octahydrophenanthrene is oxidized with potassium permanganate to manufacture the target product 1,2,3,4-benzenetetracarboxylic acid.

Preferably, the oxidation is performed in an aqueous reaction medium. The term "aqueous reaction medium", as used herein, refers to a reaction medium mainly composed of water. Thus, this term includes water that contains another solvent or dispersion medium, as well as pure water, provided that the oxidation reaction is not inhibited. Examples of the aqueous reaction medium include water and reaction media that are widely used in oxidation with potassium permanganate. Preferably, water is used as the main reaction solvent.

The reaction (oxidation) temperature depends on the amount of raw materials, the amount of reaction medium (also referred to as medium), the type of medium, and the characteristics of an apparatus and is preferably higher than the temperature at which a reaction mixture is solidified and no reaction proceeds. Preferably, the reaction (oxidation) temperature is lower than the temperature at which the medium or the raw materials are lost in large quantities by vaporization. The reaction (oxidation) temperature more preferably ranges from 10°C to 120°C and still more preferably from 40°C to 100°C. The reaction time depends on the reaction temperature, the amount of raw materials, the amount of medium, and the agitation efficiency and ranges from about 2 to 48 hours. Even when heating and agitation are stopped to interrupt the reaction, heating and agitation may be subsequently resumed to restart the reaction.

The molar ratio of 1,2,3,4,5,6,7,8-octahydrophenanthrene to potassium permanganate preferably ranges from 1:8 to 1:30 and more preferably from 1:12 to 1:22. At least eight times the molar amount of potassium permanganate results in small quantities of remaining reaction intermediates and unreacted raw materials, a high yield of the target product, and easy purification. Not more than 30 times the molar amount of potassium permanganate results in high reactor efficiency, efficient consumption of potassium permanganate, and less side reactions.

Potassium permanganate may be added to a reaction system at a time at the beginning of the reaction or in several portions. To avoid using an excessive amount of potassium permanganate, potassium permanganate may be gradually added to a reaction system in the final stage of the reaction on the basis of the analysis of the color (characteristic purple) of the reaction system and the proportion of residual raw materials or reaction intermediates.

The mass ratio of potassium permanganate to the reaction medium preferably ranges from 1:2 to 1:50 and more preferably from 1:3 to 1:24. A mass ratio of 1:2 or more results in high agitation efficiency and efficient dissolution or dispersion of potassium permanganate, thus enhancing the function of potassium permanganate as an oxidizing agent. A mass ratio of 1:50 or less results in high reactor efficiency and reduced reaction time.

If necessary, a reaction product is purified. As described in J. Am. Chem. Soc. 1952, 74, 116 or Pharmaceutical Society of Japan 1953, 73, 928, a target product can be obtained as a barium salt of carboxylic acid, subjected to acidity adjustment and neutralization to increase the purity, and purified by recrystallization in water or acid. Purification methods described in examples of the present application are also suitably used. More specifically, the purity can be increased by a process that includes, in order of application, (1) quenching excess potassium permanganate in a reaction mixture using methanol, (2) removing insoluble matter, such as manganese dioxide, by filtration, (3) neutralizing the resulting filtrate with an acid, (4) drying the neutralized solution into a crude containing a high proportion of salt and extracting a target product with acetic acid, and (5) evaporating the acetic acid extract and then recrystallizing 1,2,3,4-benzenetetracarboxylic acid in diluted hydrochloric acid.

When a medium in which 1,2,3,4,5,6,7,8-octahydrophenanthrene is insoluble, such as water, is used as the reaction medium, an additive agent, such as a surfactant, is effectively used to promote the reaction. In a reaction in such a heterogeneous system, the agitation speed and the agitation efficiency have large effects on the reaction rate.

1,2,3,4-benzenetetracarboxylic acid manufactured by the method for preparation described above is useful as a raw material for functional polymers or as an intermediate of various chemicals.

### EXAMPLES

The present invention will be more specifically described in the following examples. However, the present invention is not limited to these examples.

### EXAMPLE 1

### Synthesis of 1,2,3,4-benzenetetracarboxylic acid (1)

80.6 g (0.510 mol) of potassium permanganate and 721 g of distilled water were placed in a 1-L three-neck flask equipped with a condenser tube and were heated to 75°C with stirring to dissolve potassium permanganate. 10.0 g (0.0537 mol, GC purity of 97.1%) of 1,2,3,4,5,6,7,8-octahydrophenanthrene was added to the potassium permanganate solution for two minutes to initiate the reaction. The reaction temperature was maintained in the range of 75°C to 80°C during the reaction. As the reaction proceeded, the amount of dark brown insoluble matter (manganese dioxide) increased, and the pH increased.

8.6 hours after the beginning of the reaction (6.5 hours in the first day and restarted in the second day), 29.4 g (0.186 mol) of potassium permanganate, and 8.8 g of distilled water were added to the reaction mixture to allow the reaction to proceed. 102 g (0.28 mol) of 10% hydrochloric acid was added for a period of from 14.2 to 15 hours after the beginning of the reaction, and the pH was reduced from a little more than 9 to a little more than 7. The reaction was completed in 19.3 hours.

After cooling the reaction mixture, 4.15 g (0.130 mol) of methanol was added to the reaction mixture. The reaction mixture was stirred at room temperature until the purple color of the liquid layer disappeared (about two hours) and was then subjected to suction filtration to remove a solid. The filtrate was again filtered to remove a very small amount of remaining insoluble matter and was then concentrated to about 200 mL under reduced pressure (at this point, the pH was a little less than 9). 17.1 g (0.169 mol) of concentrated hydrochloric acid was added to the concentrate at room temperature with stirring for 1.4 hours to neutralize the concentrate (a solid precipitated out of the concentrate during the neutralization, but the concentrate finally became a homogeneous solution having a pH in the range of 1 to 2). After the filtrate was filtered again, the filtrate was dried under reduced pressure to yield 37.9 g of light flesh-colored solid.

37.1 g of the resulting crude and 261.8 g of acetic acid were placed in a 300-mL flask and were stirred under reflux for 3 hours. After insoluble matter (white solid) was removed by hot filtration, the resulting filtrate was dried to yield 7.73 g of solid. 52.3 g of 13.4% hydrochloric acid was added to the solid. The mixture was stirred under reflux and was cooled to 0°C to promote crystallization. 4.68 g of wet solid (LC purity of about 96%) was collected by filtration. 66.8 g of 15.0% hydrochloric acid was added to the wet solid in a 100-mL flask. The wet solid was dissolved with stirring under reflux. After hot filtration, the filtrate was cooled to room temperature with stirring for crystallization. 3.46 g wet solid was collected by filtration. The wet solid was dried under reduced pressure to yield 3.21 g of target product 1,2,3,4-benzenetetracarboxylic acid (LC purity of at least 97%, yield 23%).

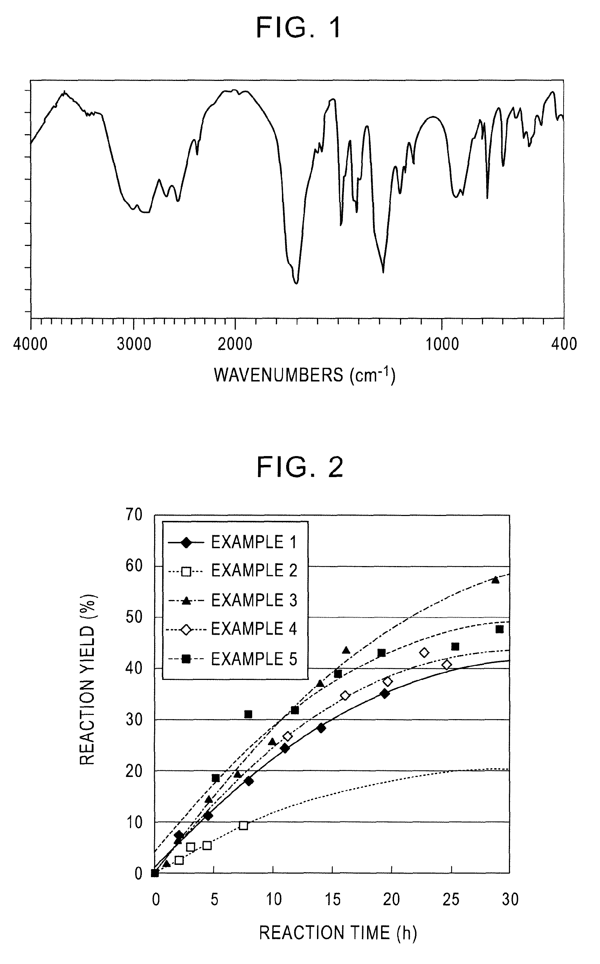

The results of analysis of 1,2,3,4-benzenetetracarboxylic acid were as follows: NMR: ¹H NMR (400 MHz, DMSO-d₆) δ (ppm) 13.2 - 13.7 (b, 4H, -COOH), 7.91 (s, 2H, ArH); Elementary analysis: Calc'd for C₁₀H₆O₈: C = 47.26%, H = 2.38%, O = 50.36%; Found: C = 47.19%, H = 2.33%, O = 49.88%; IR: see Fig. 1. These results agreed with the structure of 1,2,3,4-benzenetetracarboxylic acid.

The results of analysis of a product prepared by dehydrating the carboxy groups of 1,2,3,4-benzenetetracarboxylic acid with acetic anhydride were as follows: Melting point: about 198.4°C; NMR: ¹H NMR (400 MHz, DMSO-d₆) δ (ppm) 8.57 (s, 2H, ArH); Elementary analysis: Calc'd for C₁₀H₂O₆: C = 55.06%, H = 0.92%, O = 44.01%; Found: C = 54.78%, H = 0.98%, O = 44.22%. These results agreed with the structure of 1,2,3,4-benzenetetracarboxylic dianhydride. This also demonstrated the formation of 1,2,3,4-benzenetetracarboxylic acid.

### EXAMPLE 2

### Synthesis of 1,2,3,4-benzenetetracarboxylic acid (2)

230.0 g (1.455 mol) of potassium permanganate and 1.60 kg of distilled water were placed in a 2-L three-neck flask equipped with a condenser tube, a mechanical stirrer, and a thermometer and were heated to 73.5°C with stirring to dissolve potassium permanganate. 22.98 g (0.1234 mol) of 1,2,3,4,5,6,7,8-octahydrophenanthrene was added to the solution for one minute to initiate the reaction. The reaction temperature was maintained in the range of 75°C to 80°C, and the agitation speed was about 200 rpm. As the reaction proceeded, the amount of dark brown insoluble matter increased, and the pH increased. 134.4 g (0.398 mol) of 10.8% hydrochloric acid was added for a period of from 5.1 to 5.7 hours after the beginning of the reaction, and the pH was reduced from a little more than 8 to a little more than 7. The reaction was carried out for 7.5 hours in total.

After cooling the reaction mixture, 27.1 g (0.844 mol) of methanol was added to the reaction mixture. The reaction mixture was stirred at about 40°C for 6 hours and was then subjected to suction filtration to remove a solid. After the filtrate was concentrated under reduced pressure to about 540 g, the concentrate was washed with 60 g of toluene to remove oil. 144 g (0.427 mol) of 10.8% hydrochloric acid was added to the concentrate at room temperature with stirring for 0.8 hours to neutralize the concentrate. The reaction product was filtrated to remove a minute amount of solid and was then dried under reduced pressure to yield 63.1 g of light flesh-colored solid (which was subsequently purified as in Example 1).

### EXAMPLE 3

### Synthesis of 1,2,3,4-benzenetetracarboxylic acid (3)

233.9 g (1.480 mol) of potassium permanganate and 1.54 kg of distilled water were placed in a 2-L three-neck flask equipped with a condenser tube, a mechanical stirrer, and a thermometer and were heated to 74°C with stirring to dissolve potassium permanganate. 24.88 g (0.1336 mol) of 1,2,3,4,5,6,7,8-octahydrophenanthrene was added to the solution for two minutes. 0.092 g of surfactant (Mama Lemon manufactured by Lion Corporation) was added to the solution to initiate the reaction. 0.063 g of the surfactant was added 5.3 hours after the beginning of the reaction.

The reaction temperature was maintained in the range of 75°C to 85°C, and the agitation speed ranged from about 280 to 350 rpm. As the reaction proceeded, the amount of dark brown insoluble matter increased, and the pH increased. After the beginning of the reaction, 21.1 g (0.134 mol) of potassium permanganate and 68.1 g of distilled water were added at 12.9 hours, 42.3 g (0.268 mol) of potassium permanganate and 113.3 g of distilled water were added at 14.7 hours, 22.1 g (0.140 mol) of potassium permanganate and 21.8 g of distilled water were added at 17.9 hours, 21.1 g (0.134 mol) of potassium permanganate and 29.2 g of distilled water were added at 20 hours, and 21.1 g (0.134 mol) of potassium permanganate and 84.3 g of distilled water were added at 23.3 hours to allow the reaction to proceed. The reaction was completed in 28.5 hours (8.9 hours in the first day, 11.1 hours in the second day, and 8.5 hours in the third day).

After cooling the reaction mixture with water, 16.1 g (0.503 mol) of methanol was added to the reaction mixture. The reaction mixture was stirred at room temperature for 1.5 hours, was left still for 12 hours, and was subjected to suction filtration to remove a solid. The filtrate was concentrated under reduced pressure to about 260 g. 118.8 g (1.173 mol) of concentrated hydrochloric acid was added to the concentrate at room temperature with stirring for one hour to neutralize the concentrate. The reaction mixture was dried under reduced pressure to yield 132.7 g of light-colored solid (which was subsequently purified as in Example 1).

### EXAMPLE 4

### Synthesis of 1,2,3,4-benzenetetracarboxylic acid (4)

237.0 g (1.50 mol) of potassium permanganate and 1.37 kg of distilled water were placed in a 2-L three-neck flask equipped with a condenser tube, a mechanical stirrer, and a thermometer and were heated to 73.6°C with stirring to dissolve potassium permanganate. 28.75 g (0.1543 mol) of 1,2,3,4,5,6,7,8-octahydrophenanthrene was added to the solution for 1.5 minutes to initiate the reaction. 0.098 g of surfactant (Mama Lemon manufactured by Lion Corporation) was immediately added to the solution. 0.063g of the surfactant was further added to the solution 17.7 hours after the beginning of the reaction. The reaction temperature was maintained in the range of 76°C to 84°C, and the agitation speed was about 350 rpm.

As the reaction proceeded, the amount of dark brown insoluble matter increased, and the pH increased. After the beginning of the reaction, 79.9 g (0.506 mol) of potassium permanganate was added at 11 hours, 49.3 g (0.312 mol) of potassium permanganate was added at 17.5 hours, and 520 g of distilled water was added at 23 hours to allow the reaction to proceed. The reaction was completed in 24.5 hours (10.5 hours in the first day, 12.5 hours in the second day, and 1.5 hours in the third day).

After cooling the reaction mixture, 23.6 g (0.737 mol) of methanol was added to the reaction mixture. The reaction mixture was stirred at room temperature for 6 hours and was then subjected to suction filtration to remove a solid. The filtrate was concentrated under reduced pressure to about 680 g. 282.8 g (2.792 mol) of concentrated hydrochloric acid was added to the concentrate at room temperature with stirring for 0.4 hours. The concentrate was then stirred under reflux for 3.5 hours to promote neutralization. The reaction mixture was dried under reduced pressure to yield 124.5 g of cream solid (which was subsequently purified as in Example 1).

### EXAMPLE 5

### Synthesis of 1,2,3,4-benzenetetracarboxylic acid (5)

20.5 g (0.130 mol) of potassium permanganate and 187.4 g of distilled water were placed in a 300-mL three-neck flask equipped with a condenser tube and a thermometer and were heated to about 70°C with stirring to dissolve potassium permanganate. 0.021 g of surfactant (kitchen detergent manufactured by Lion Corporation) and 1.862 g (0.0100 mol) of 1,2,3,4,5,6,7,8-octahydrophenanthrene were added to the solution to initiate the reaction. The solution was immediately heated to a reflux temperature to allow the reaction to proceed. As the reaction proceeded, the amount of dark brown insoluble matter increased, and the pH increased.

After the beginning of the reaction, 4.76 g (0.0301 mol) of potassium permanganate and 40.1 g of distilled water were added at 8 hours, and 1.58 g (0.0100 mol) of potassium permanganate and 11.9 g of distilled water were added at 19 hours to allow the reaction to proceed. The reaction was completed in 29 hours (8 hours in the first day, 11 hours in the second day, and 10 hours in the third day).

After cooling the reaction mixture, 1.0 g (0.031 mol) of methanol was added to the reaction mixture. The reaction mixture was stirred at room temperature for 1.5 hours and was then subjected to suction filtration to remove a solid. The filtrate was concentrated under reduced pressure to 96 g. 26.0 g (0.257 mol) of concentrated hydrochloric acid was added to the concentrate at room temperature with stirring for 0.3 hours, and the concentrate was then stirred under reflux for 2.9 hours to neutralize the concentrate. The reaction mixture was dried under reduced pressure to yield 8.69 g of light-colored solid (LC analysis showed that about 1.1 g of the solid was the target product. Purification was subsequently performed as in Example 1).

Fig. 2 is a graph illustrating the reaction yield as a function of reaction time in Examples 1 to 5. The data points in Fig. 2 represent actual measurements, and the curved lines are auxiliary lines. The reaction yield was calculated from the amount of the target product in the reaction system by LC analysis.

### EXAMPLE 6

### Synthesis of 1,2,3,4-benzenetetracarboxylic acid (6)

4.28 g (27.1 mmol) of potassium permanganate and 82.0 g of distilled water were placed in a 300-mL three-neck flask equipped with a condenser tube and were stirred at room temperature to dissolve potassium permanganate. 0.4387 g (2.355 mmol) of 1,2,3,4,5,6,7,8-octahydrophenanthrene was added to the solution and was allowed to react at room temperature for 4.1 hours. The solution was then heated to 49°C to allow the reaction to proceed for another 1.9 hours. As the reaction proceeded, the amount of dark brown insoluble matter increased.

After cooling the reaction mixture, methanol was added to the reaction mixture to quench excess potassium permanganate. After removing insoluble matter, LC analysis of the filtrate showed that the filtrate contained 1,2,3,4-benzenetetracarboxylic acid.

### EXAMPLE 7

### Synthesis of 1,2,3,4-benzenetetracarboxylic acid (7)

4.58 g (29.0 mmol) of potassium permanganate, 85.3 g of distilled water, and 2.76 g (30.4 mmol) of concentrated nitric acid (69% to 70%) were placed in a 300-mL three-neck flask equipped with a condenser tube and were heated to 80°C with stirring. 0.3652 g (1.960 mmol) of 1,2,3,4,5,6,7,8-octahydrophenanthrene was added to the mixture. The mixture was heated to a reflux temperature to allow the reaction to proceed for 4 hours.

After cooling the reaction mixture and removing insoluble matter, LC analysis of the filtrate showed that the filtrate contained a small amount of 1,2,3,4-benzenetetracarboxylic acid. This is probably because a decomposition reaction was dominant at high temperature under acidic conditions, thus reducing the yield.

### EXAMPLE 8

### Synthesis of 1,2,3,4-benzenetetracarboxylic acid (8)

1,2,3,4-benzenetetracarboxylic acid with a purity of about 99% was prepared as in Example 1 except that 1,2,3,4,5,6,7,8-octahydrophenanthrene with a GC purity of 98.9% was used.

As much as about 4% by mass of phenanthrene is contained in coal tar, and isolation techniques of phenanthrene have been established. It is also known that 1,2,3,4,5,6,7,8-octahydrophenanthrene used in the present invention can be manufactured by the hydrogenation reaction of phenanthrene in a single step. Thus, there are no quantitative or technical constraints on the raw material supply, and the reaction involves only two steps. In particular, oxidation of 1,2,3,4,5,6,7,8-octahydrophenanthrene with potassium permanganate can proceed under a mild reaction condition of atmospheric pressure and can therefore be performed in general-purpose facilities without using special facilities, such as facilities with acid resistance and high-pressure resistance, causing less problems from a safety and environmental standpoint.

Furthermore, in scale-up, oxidation of 1,2,3,4,5,6,7,8-octahydrophenanthrene with potassium permanganate has no disadvantage associated with conventional methods and has an advantage over the conventional methods.

### Industrial Applicability

According to the present invention, 1,2,3,4-benzenetetracarboxylic acid, which is useful as a raw material for functional polymers or as an intermediate of various chemicals, can be manufactured with an advantage over conventional methods. More specifically, the present invention is superior to conventional methods in that it requires no special facilities, such as facilities with acid resistance and high-pressure resistance, involves only a two-step process, and causes less problems from a safety and environmental standpoint. In particular, the present invention has an advantage over conventional methods in scale-up.

## Claims

1. A method for preparation of 1,2,3,4-benzenetetracarboxylic acid, comprising the step of oxidizing 1,2,3,4,5,6,7,8-octahydrophenanthrene with potassium permanganate.

2. The method for preparation according to Claim 1, wherein the oxidation is performed in an aqueous reaction medium.

3. The method for preparation according to Claim 1, wherein the oxidation is performed under alkaline conditions.

4. The method for preparation according to Claim 1, wherein the oxidation is performed at a temperature in the range of 10°C to 120°C.

5. The method for preparation according to Claim 1, wherein the oxidation is performed at a molar ratio of 1,2,3,4,5,6,7,8-octahydrophenanthrene to potassium permanganate in the range of 1:8 to 1:30.

6. The method for preparation according to Claim 2, wherein the oxidation is performed at a mass ratio of potassium permanganate to the aqueous reaction medium in the range of 1:2 to 1:50.

7. The method for preparation according to any one of Claims 1 to 6, further comprising the step of extracting 1,2,3,4-benzenetetracarboxylic acid with acetic acid and purifying 1,2,3,4-benzenetetracarboxylic acid after the oxidation reaction.
